# EUROPEAN PATENT SPECIFICATION

(11) **EP 0 422 186 B1**
(45) Date of publication and mention of the grant of the patent: **09.12.1998**
(21) Application number: 90906668.0
(22) Date of filing: 09.04.1990
(51) Int. Cl.: A61K 38/00

(54) **USE OF ONCOSTATIN M FOR SUPPRESSING MHC ANTIGENS**
VERWENDUNG VON ONCOSTATIN M ZUR SUPPRESSION VON MHC ANTIGENEN
UTILISATION DE L'ONCOSTATINE M POUR LA SUPPRESSION DES ANTIGENES MHC

(30) Priority: 10.04.1989 US 335399; 04.04.1990 US 504486
(43) Date of publication of application: 17.04.1991
(73) Proprietor: ONCOGEN LIMITED PARTNERSHIP, Seattle, WA 98121 (US)
(72) Inventor: BROWN, Thomas, J., Poulsbo, WA 98370 (US); GLADSTONE, Paul, R., Seattle, WA 98117 (US)
(74) Representative: Hirsch, Marc-Roger
(86) International application number: US9001907
(87) International publication number: WO9012585

(56) References cited:
- EP-A- 290 948
- J. CELL. BIOCHEM. vol. 0, no. 13-E, 1989, page 189 T. BROWN ET AL 'Oncostatin M as a unique modulator of endothelial cell surface properties'
- J. CELL. BIOCHEM. vol. 0, no. 12-A, 1988, page 224 N. GUNDERSON ET AL 'Expression of oncostatin m and properties of the recombinant protein.'
- PROC. NATL. ACAD. SCI. vol. 83, no. 24, 1986, pages 9739 - 9743 J.M. ZARLING ET AL 'Oncostatin M: a growth regulator produced by differentiated histiocytic lymphoma cells'
- Molecular and Cellular Biology (United States), Volume 10(5), May 1990. P.S. LINSLEY et al., "Cleavage of a Hydrophilic C-Terminal domain Increases Growth-Inhibitory Activity of Oncostatin M." pages 1882-90. see Abstract.
- Journal of Immunology, Volume 139(9), 01 November 1987, T.J. BROWN et al., "Purification and Characterization of Cytostatic Lymphokines Produced by Activated Human T Lymphocytes." pages 2977-2983.
- Proceedings of the Nation Academy of Sciences (USA), Volume 83(24), December 1986, J.M. ZARLING et al., "Oncostatin M as a Growth Regulator Produced by Differentiated Histiocytic Lymphoma Cells", pages 9739-43. see Abstract (BIOSIS No. 830 76929).
- Molecular and Cellular Biology, Vol. 9, No. 7, July 1989. N. MALIK et al. "Molecular Cloning, Sequence Analysis, and Functional Expression of a Novel Growth Regulator, Oncostatin M". pages 2847-2853.
- Journal of Cellular Biochemistry, Supplement, Vol. 0(12), 1988 P.S. LINSLEY et al. "Indentification of a Membrane Receptor for the Growth Regulator, Oncostatin M", page 227. see Abstract.
- Journal of Cellular Biochemistry Supplement, Vol. 0(12, Part A). 1988 T.J. BROWN, et al. "Oncostatin M Differentially Regulates the Action of Type 1 Transforming Growth Factor-beta (TGF-beta 1)", page 194. see Abstract.
- Journal of Cellular Biochemistry, Supplement. Vol. 0(12 Part A), 1988. N. GUNDERSON, et al., "Expression of Oncostatin on and Properties of the Recombinant Protein", page 224. see Abstract.
- Journal of Cellular Biochemistry, Supplement. Vol. 0(13 Part E) 1988. T BROWN, et al. "Oncostatin Masa Unique Modulator of Endothelial Cell Surface Properties", page 189. see Abstract.

## Description

The present invention is directed to the use of a recently discovered cytokine, Oncostatin M, for inhibiting endothelial tissue immunogenicity and for improving the likelihood of transplanted organ functional success. The method of the invention includes the use of mature, hybrid, modified or truncated forms of Oncostatin M as well as Oncostatin M analogs. The invention is described by way of examples in which the efficacy of such compounds is evaluated using various in vitro assay systems.

### 2. BACKGROUND OF THE INVENTION

### EXPRESSION OF MAJOR HISTOCOMPATIBILITY COMPLEX ANTIGENS AND RELATIONSHIP TO IMMUNE RESPONSE

Surface-expressed proteins of the major histocompatibility complex (MHC) are integral for the initiation and effector functions of immune responses. Initiation of immune functions by CD4+ T cells (helpers) appear to require class II MHC antigen presentation, while cytotoxic effector functions by CD8+ T cells (cytotoxic T lymphocytes or CTLs) appear to require class I MHC antigen presentation. It is known that cell surface expression of MHC proteins correlates with human autoimmune and alloimmune diseases (reviewed in Feldman et al., in Interferon 9: 75-90, Academic Press 1987). Tissue cells in the majority of human autoimmune diseases including, for example, thyroiditis, diabetes, multiple sclerosis, systemic lupus erythematosus, rhematoid arthritis, Sjogren's syndrome, vasculitis, bilary cirrhosis and immunologically-related skin disorders, as well as abnormally elevated levels of class I and class II MHC antigens. When cells that ordinarily do not express class II antigens (e.g., fibroblasts and endothelial cells) begin to express this class of antigen, they become immunoreactive targets for lysis and destruction by CTLs (Pober et al., 1983, Nature 305: 726).

MHC antigen expression on many cell types is largely under the regulatory control of various cytokines. Cells such as macrophages, dermal fibroblasts, keratinocytes, thyrocytes, astrocytes, B islet cells, smooth muscle cells, T lymphocytes, endothelial cells and many cancer cells, require induction by cytokines for class II MHC antigen expression (Revel and Schattner, in Autoimmunity and Autoimmune Disease 223-233, Wiley 1987). Members of the interferon family are primarily involved with the upregulation of MHC expression and all types of interferons appear to enhance class I expression, although IFN-γ is the best studied and most potent in this regard. In addition, the cytotoxins known as tumor necrosis factors (TNF-α and TNF-β) have demonstrated the ability to enhance class I expression in fibroblasts via induction of IFN-β2 (May et al, 1987, Proc. Natl. Acad Sci. USA 83: 8957). While the induction of class II antigens by IFN-α and IFN-β is usually not significant, IFN-γ is a potent inducer of class II molecules at the gene level (Collins et al., 1984, Proc. Natl Acad. Sci. USA 81: 4917). Some cytokines appear to synergize with IFN-γ in the induction of MHC antigen expression; for example, TNF-α and TNF-β act synergistically with IFN-γ on expression of class I antigens on endothelial cells without affecting IFN-γ-mediated induction of class II antigens (Lapierre et al., 1988, J. Exp. Med. 167: 794).

The level of MHC antigen expression on a cell's surface is a determinant of its antigen-presenting capacity (Matis et al., 1983, Proc. Natl. Acad. Sci. USA 80: 6090). Therefore, compounds that interfere with or antagonize over-expression of MHC antigens beyond immunologically-tolerated thresholds may have therapeutic utility in, for example, slowing or stopping the progression of autoimmune diseases. In the case of transplanted organs, the expression level of the donor MHC antigens is an important determinant of the severity of the rejection response (Ferry et al., 1987, Transplantation 44: 499). Transforming Growth Factor-β (TGF-β) has been shown to inhibit the level of class II expression induced by IFN-γ on human melanoma cells and peripheral blood mononuclear cells without affecting class I expression (Czarniecki et al., 1988, J. Immunol. 140: 4217).

### 2.1.2. ENDOTHELIAL CELL EXPRESSION OF MHC ANTIGENS: RELATIONSHIP TO ALLOGRAFT REJECTION

Vascular endothelial cells play a central role in the process of allograft rejection. Immunohistologic studies of tissue rejection have demonstrated that vascular endothelial cells from MHC-incompatible heart or skin express high levels of class II MHC antigens early in the rejection process (De Waal et al., 19883, Nature 303: 426-429; Milton and Fabre, 1985, J. Exp. Med. 161: 98-112). In its inception, immunologic rejection of allotransplantations involves the interaction between recipient T cells and the donor organ vasculature; other cells become involved as rejection progresses. In organ transplants the endothelium is the donor tissue which is the first target of attack by host T cells recognizing doner HLA antigens on the surface of endothelial cells. T cell infiltration through the vascular endothelium has been described as a four step process: recognition, adherence, activation, and penetration of alloactivated T cells through the vascular wall (Fung et al.,1986, Human Immunol. 16: 182). Activated T cells produce gamma-interferon (IFN-γ) which stimulates the expression of class II HLA antigens on the surface of the donor endothelium, thereby transforming the donor endothelium to a more immunogenic state. Increased expression of class I antigens also results from induction by gamma-IFN and TNFs, probably augmenting the target function of the endothelium in interactions with cytotoxic T lymphocytes. In early biopsies of rejected kidney grafts, T cells with a specificity for class I HLA antigens are predominant; as rejection progresses, T cells specific for class II HLA antigens are prevalent (Zeevi et al., 1986, Transplantation 41: 620). Similar results have been found in heart transplants. The degree of alloimmune response and, ultimately, rejection depends on the expression of both class I and class II HLA molecules, and even small reductions in their expression levels leads to substantially improved outcomes.

Endothelial cells provide a good model for studying cytokine regulation of MHC expression in vitro since these cells are readily available from primary tissue and are known to function as antigen presenting cells following treatment with cytokines. Cultured human umbilical endothelial cells (HUVECs) constitutively express class I but not class II MHC (HLA) antigens under normal culture conditions. Alpha and beta interferons (IFN-α, IFN-β), as well as alpha and beta tumor necrosis factors (TNF-α, TNF-β), increase the levels of class I HLA expression in HUVECs without affecting class II HLA levels (Collins et al., 1986, Proc. Natl. Acad. Sci. USA 38: 446; Pober et al., 1987, J. Immunol. 138: 3319). Only gamma interferon(IFN-γ) demonstrates the capacity to upregulate both class I and II HLA expression (Geppert and Lipsky, 1985, J. Immunol. 135: 3750). Cytokine treatment of HUVECs results in increased levels of steady-state RNA levels for HLA antigens as well as increased cell-surface expression (Collins et al., 1986, Proc. Natl. Acad. Sci. USA 38: 446). TNF-α and TNF-β act synergistically with IFN-γ to enhance class I MHC expression without affecting IFN-γ-mediated class II induction (Lapierre et al., 1988, J. Exp. Med. 167: 794). In contrast, neither IFN-α nor IFN-β synergize with IFN-γ to enhance class I expression, but both inhibit the IFN-γ-mediated induction of class II expression.

### 3. SUMMARY OF THE INVENTION

The use of Oncostatin M to control endothelial cell immunogenicity, and improve the likelihood of transplanted organ functional success is described. Oncostatin M purified from natural sources, recombinant Oncostatin M, or Oncostatin M prepared by chemical synthetic techniques may be used. The invention is described by way of examples in which the various effects of Oncostatin M on several cell types are determined using in vitro assays.

The invention is directed to the use of Oncostatin M to mitigate endothelial tissue immunogenicity, the primary element involved in the rejection of allografted organs and the progression of certain autoimmune diseases. This aspect of the invention is described by way of examples in which the ability of Oncostatin M to inhibit the expression of MHC antigens on the surface of human endothelial cells in vitro is demonstrated. Oncostatin M is capable of substantially antagonizing IFN-γ and TNF-α stimulated expression of class I and class II HLA antigens and appears to be specific for action on endothelial cells.

### 4. DESCRIPTION OF THE FIGURES

FIG. 1. Synergistic inhibitory effects of Oncostatin M and TGF-β on endothelial cell HLA antigen expression induced by IFN-γ. Oncostatin M ( ); TGF-β ( ); Oncostatin M plus TGF-β ( ). A: Effects on class I HLA antigen expression. B: Effects on class II HLA-DR antigen expression.

FIG. 2. Oncostatin M receptor binding on HUVECs: saturation curve, scatchard plot and SDS-PAGE autoradiograph (inset). Data obtained and compiled as described in Section 6.2.4., infra.

### 5. DETAILED DESCRIPTION OF THE INVENTION

The present invention is directed to the use of Oncostatin M for the manufacture of a medicament for inhibiting endothelial tissue immmunogenicity, whereby the expression of MHC antigens on the endothelial cells is suppressed, as defined in claims 1 to 10. The present invention also concerns the use of Oncostatin M for the manufacture of a medicament for improving the likelihood of transplanted organ functional success, whereby the expression of MHC antigens on donor organ vasculature is inhibited, as defined in claims 11 to 24.

The invention is based in part on the discovery that Oncostatin M mediates various biological effects on endothelial cells. The invention is described by way of examples in which the various biological effects of Oncostatin M on mammalian cells are determined using in vitro assay systems. The clinical implications of the biological effects mediated by Oncostatin M revealed and described herein may encompass a wide range of therapeutic uses of Oncostatin M pertaining to human immunology

Oncostatin M, originally identified for its inhibitory effects on human tumor cell lines, was first isolated from phorbol 12-myristate 13-acetate (PMA)-induced human histiocytic lymphoma cells (Zarling et al., 1986, Proc. Natl. Acad. Sci. USA 83: 9739-9743) and from activated T lymphocytes (Brown et al., 1987, J. Immunol. 139: 2977-2983). The molecule is a heat and acid stable protein comprised of a single polypeptide chain of Mᵣ = 28,000. Like other naturally occurring growth regulators, Oncostatin M exhibits a variety of biological activities. Growth inhibition is observed with some, but not all, human tumor cell lines. In contrast, the growth of some normal fibroblasts, such as human foreskin fibroblasts or WI-38 cells, is stimulated by exposure to Oncostatin M (Zarling et al., 1986, Proc. Natl. Acad. Sci. USA 83: 9739-9743). The gene for Oncostatin M has been cloned and sequenced, and an active form of recombinant Oncostatin M has recently been expressed in mammalian cells (Copending application Serial No.144,574 filed January 15, 1988, which is incorporated herein by reference in its entirety). The mature form, after cleavage of the signal peptide, is a glycoprotein containing 228 amino acids, five of which are cysteine residues. The protein has an extremely hydrophilic carboxy terminal domain. Although Oncostatin M is not structurally related to other known cytokines, its mRNA contains an AU-rich region at its 3' untranslated end. This region in the Oncostatin M message is homologous to that of many cytokines, lymphokines and other growth-regulatory molecules, suggesting a common mode of regulating gene expression. A cellular receptor for Oncostatin M has been found on a variety of mammalian cells. The major Oncostatin M receptor molecule is a specific protein of Mr = 150,000-160,000 (Linsley et al, 1989, J. Biol. Chem. 264: 6528-6532).

In accordance with the invention, Oncostatin M may be obtained by techniques well known in the art from a variety of cell sources which synthesize bioactive Oncostatin M including, for example, cells which naturally produce Oncostatin M and cells transfected with recombinant DNA molecules capable of directing the synthesis and/or secretion of Oncostatin M. Alternatively, Oncostatin M may be synthesized by chemical synthetic methods including but not limited to solid phase peptide synthesis Methods for the production of Oncostatin M are described in copending application Serial No. 144,574 filed January 15, 1988, a continuation-in-part of application Serial No. 046, 846 filed May 4, 1987, a continuation-in-part of application Serial No. 935,283 filed November 26, 1986, a continuation-in-part of application Serial No. 811,235 filed December 20, 1985, each of which is incorporated by reference herein in its entirety.

In the practice of the method of the invention, the use of Oncostatin M obtained by any method, as well as the use of modified or truncated Oncostatin molecules and Oncostatin M analogs which retain the desired activity, are within the scope of the invention. In this regard, variations in the Oncostatin M primary structure, as well as variations in higher levels of structural organization, the type of covalent bonds linking the amino acid residues, and/or addition of groups to the terminal residues of Oncostatin M are within the scope of the invention. For example, the Oncostatin M molecule used in accordance with the invention may include conservative or non-conservative alterations in the amino acid sequence which result in silent changes that preserve the functionality of the molecule including, for example, deletions, additions and substitutions. Such altered Oncostatin M molecules may be desirable where they provide certain advantages in their use. As used herein, conservative substitutions would involve the substitution of one or more amino acids within the sequence of Oncostatin M with another amino acid having similar polarity and hydrophobicity/hydrophilicity characteristics resulting in a functionally equivalent molecule. Such conservative substitutions include but are not limited to substitutions within the following groups of amino acids: glycine, alanine; valine,isoleucine, leucine; aspartic acid, glutamic acid; asparagine, glutamine; serine, threonine; lysine, arginine; phenylalanine, tyrosine; and methionine, norleucine.

In another embodiment of the invention, Oncostatin M may be linked to carrier molecules. For example, Oncostatin M could be covalently coupled to an antibody molecule specific for endothelial cells or for some other cell surface antigen which will allow Oncostatin M to be targeted to cells which express that particular antigen. Similarly, Oncostatin M may be linked to other "targeting" molecules such as hormones, growth factors, cytokines, etc. In this way, the Oncostatin M molecule could be altered so that it is taken up by cells that may not express a receptor for the particular Oncostatin M molecule chosen for use. Such coupling techniques are well known in the art and can include, for example, the use of cross-linking agents, schiff-base formation, amide bonds, peptide bonds, sulfide bonds, etc.

### 5.1. ONCOSTATIN M AS AN IMMUNOMODULATOR

One aspect of the present invention relates to the use of Oncostatin M as an immunomodulator in the treatment of human autoimmune and alloimmune diseases. In this regard, applicants have discovered through a series of experiments such as those described in Section 6., et seq. that Oncostatin M is capable of inhibiting the expression of class I and II HLA antigens on the surface of human endothelial cells in vitro. Therefore, Oncostatin M may find use in decreasing the immunogenicity of endothelial tissue to helper and/or effector T lymphocytes.

### 5.1.1. USE OF ONCOSTATIN M IN ORGAN TRANSPLANTATION

Since class I molecules appear to be the initial target for cytotoxic T lymphocytes, depression in class I expression would be expected to prevent or inhibit the onset of strong secondary responses. Perhaps more importantly, the inhibition of class II expression on endothelial cells may block the recruitment of antigen-specific, gamma-interferon-secreting helper T lymphocytes, thus thwarting the development of immune response. In a particular embodiment of the invention, compounds containing effective doses of Oncostatin M formulated in suitable pharmacological carriers may be administered to organ transplant recipients such as kidney, heart and lung transplant recipients, via any appropriate route including but not limited to local or systemic injection, in order to inhibit or prevent the expression of HLA antigens and rejection of the transplant. In addition, Oncostatin M may be linked to a carrier or targeting molecule and/or incorporated into liposomes, microcapsules, and controlled release preparations prior to administration in vitro. Through a series of experiments, such as those described in Section 6, infra, applicants have determined that endothelial cell expression of class I and II MHC antigens, particularly gamma-interferon- and tumor necrosis factor-alpha-stimulated expression, is specifically antagonized by Oncostatin M at concentrations ranging from 1-50 ng/ml. For example, at a concentration of 5 ng/ml, Oncostatin M inhibited IFN-γ-stimulated class I antigen expression by 51% and TNF-α-stimulated expression by a striking 141%. Induction of class II antigen expression by IFN-γ was inhibited by as much as 84%. When used in combination with a synergistic cytokine such as TGF-β, low concentrations of Oncostatin M were effective in producing strong inhibitory effects. In addition, applicants' research data suggests that Oncostatin M is incapable of inhibiting class I or class II HLA antigen expression on cells of the monocyte/macrophage lineage. Use of Oncostatin M may therefore offer a better alternative to Cyclosporin A. The broad effects of Cyclosporin A, currently the principal immunosuppressive agent used in allograft transplantations, results from the inhibition of T lymphocyte proliferation generally. Oncostatin M does not have a similar effect on T cell proliferation.

In accordance with this aspect of the invention, Oncostatin M may be used alone or in combination with one or more other cytokines, growth factors or immunosuppressive agents including but not limited to TGF-β, Cyclosporin A and corticosteroids in order to increase the likelihood of a transplant recipient's acceptance of the donor organ. Applicants have in this regard observed that TGF-β acts synergistically with Oncostatin M to strongly inhibit the expression of HLA antigens on IFN-γ-stimulated endothelial cells. As little as 50 pg/ml Oncostatin M in combination with 0.5 ng/ml TGF-β resulted in a nearly 50% reduction of class I antigen expression (Section 6.2.2., infra). Furthermore, the expression of IFN-γ-stimulated class II antigens was inhibited 80% by the combination of 0.5 ng/ml Oncostatin M and 1 ng/ml TGF-β.

### 6. EXAMPLE: MODULATION OF ENDOTHELIAL CELL HLA ANTIGEN EXPRESSION BY ONCOSTATIN M IN VITRO

The in vitro experiments described below demonstrate, inter alia, that Oncostatin M inhibits the cytokine-stimulated expression of class I and class II HLA antigens on human endothelial cells, but not on monocyte-macrophages, and that TGF-β synergizes with Oncostatin M to produce this inhibitory effect.

### 6.1. MATERIALS AND METHODS

### 6.1.1. ENDOTHELIAL CELL CULTURES

Human umbilical vein endothelial cells (HUVECs) were isolated from umbilical vein as described (Wall et al., 1978, J. Cell. Physiol. 96: 203). Cells were passaged with collagenase and grown to confluence on gelatin-coated plasticware in CS-1 defined serum free medium (Cell Systems, Kirkland, WA) substituted with 50 µg/ml heparin and recombinant ECGS (Bionetics). Cells were given fresh unsupplemented media at least 12 hours prior to experimentation.

### 6.1.2. IMMUNOSTAINING

Several fluorescein-labeled monoclonal antibodies (MAbs) were employed for the detection of class I and class II HLA antigens: H1DE (Gladstone et al., Histocompatability Testing, 429, Dupont, Ed., 1984) was used for the detection of class I HLA antigens; a DR-specific antibody, VI.15 ( Gladstone et al., 1982, Proc. Natl. Acad. Sci. USA 79: 1235), was used for the detection of class II HLA-DR antigen; antibody 33.1 was used for the detection of HLA-DQ antigens and was provided by G. Marti (Marti et al., 1983, J. Exp. Med. 158: 1924-1983); HB10a was used for the detection of class II HLA-DR and HLA-DP antigens and was provided by Dr. E. Clark (Regional Primate Research Center at Washington University, Seattle, WA).

### 6.1.3. QUANTITATIVE ANTIGEN ASSAY

The expression of cell surface HLA antigens was measured essentially as described (Gladstone et al., 1982, Proc. Natl. Acad. Sci. USA 79: 1235-39) with modifications for fluorescent activated cell sorter (FACS) analysis (Basham and Merigan, 1983, J. Immunol. 130: 1492).

Briefly, 5 X 10⁴ cells per sample were immunostained and HLA antigen expression quantified by indirect immunofluorescence using a FACS analyzer. MAbs were incubated for 45 minutes in each step. Sample populations were compared by mean channel fluorescence on a four decade logarithmic scale and, where appropriate, linear fluorescence equivalents were obtained by conversion.

### 6.1.4. CYTOKINES

Recombinant Oncostatin M was prepared as described (Malik et al., 1989, Mol. Cell. Biol., in press), recombinant TGF-β as described in Gentry et al., 1987, Mol. Cell. Biol. 7: 3418), recombinant IFN-γ and TNF-α were purchased from Amgen Inc. Natural Oncostatin M was prepared as described in (Zarling et al., 1986, Proc. Natl. Acad. Sci. USA 38: 9737).

### 6.1.5. ONCOSTATIN M RECEPTOR ASSAY

Highly purified human recombinant Oncostatin M was radiolabeled by the IODO-GEN procedure (Fraker and Speck, 1978, Biochem. Biophys. Res. Comm. 80: 849) to a specific activity of 52 µCi/µg. Radioreceptor assays were conducted in situ on confluent monolayers of HUVECs grown in 24-well tissue culture plates (10⁵ cells/well) in duplicate. Monolayers were first washed twice with binding buffer (Dulbecco's MEM + 0.1% bovine serum albumin + 15 mM HEPES) and then 250 µl binding buffer containing 1 ng/ml radiolabeled Oncostatin M and variable amounts of unlabeled Oncostatin M were added to the monolayers. Cells were incubated at 23 degrees C for 3 hours to maintain steady state binding (Linsley et al., 1988, J. Biol. Chem. 264: 4282). Following the incubation period, cells were washed with cold binding buffer and solubilized in 1N NaOH. Radioactivity was detected by a gamma spectrophotometer and data plotted according to the method of Scatchard (Scatchard, 1949, Ann. N.Y. Acad. Sci. 51: 660). Non-specific binding was measured in the presence of a 400-fold molar excess of unlabeled Oncostatin M.

### 6.2. RESULTS

### 6.2.1. ONCOSTATIN M INHIBITS CYTOKINE-STIMULATED HLA ANTIGEN EXPRESSION ON HUMAN ENDOTHELIAL CELLS

The capacity of Oncostatin M to modulate the regulation of HUVEC MHC antigen expression by other cytokines was evaluated by indirect immunofluorescence and FACS quantitation as described in Materials and Methods, supra. Table I presents the effects of various cytokines on HUVEC HLA antigen expression. At a concentration of 100U/ml, both IFN-γ and TNF-α significantly affected the expression of class I antigen, amplifying expression by fivefold and twofold respectively. Additionally, IFN-γ amplified class II antigen expression by greater than sixfold. In contrast, neither TGF-β nor Oncostatin M were able to amplify class I or class II expression.

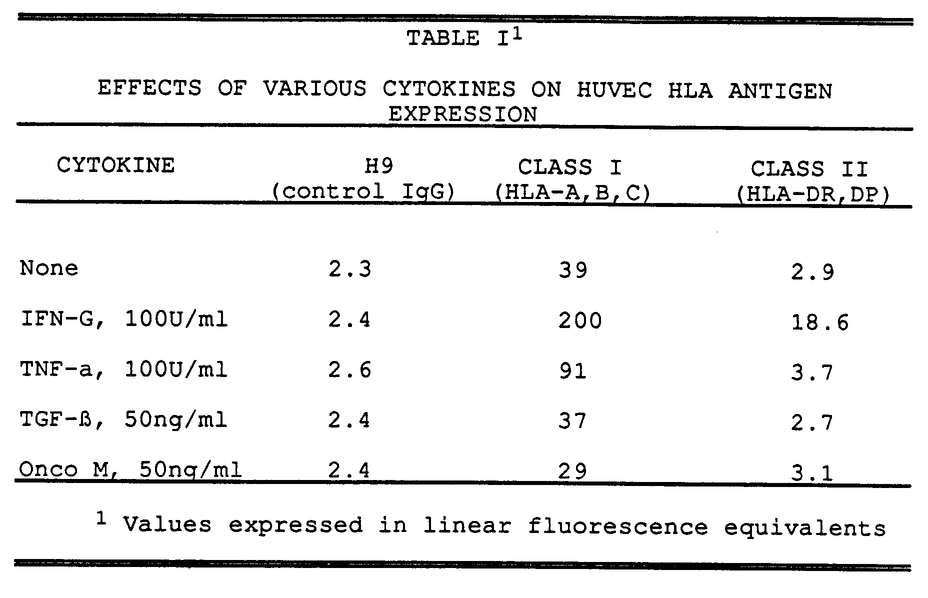

Table II presents the effects of different concentrations of Oncostatin M on the cytokine-induced expression of class I and class II antigens. Oncostatin M antagonized IFN-γ- and TNF-α-stimulated HLA antigen expression in a dose dependent manner. At a concentration of 5 ng/ml, Oncostatin M inhibited IFN-γ-stimulated class I HLA-A,B,C antigen expression by 51% and TNF-α-stimulated expression by 141%. Induction of class II HLA-DR and HLA-DQ antigens by IFN-γ was inhibited 84% and 72%, respectively, with 50 ng/ml Oncostatin M. These results strongly indicate that Oncostatin M may be useful in down-modulating the immunogenicity of endothelial cells in vivo.

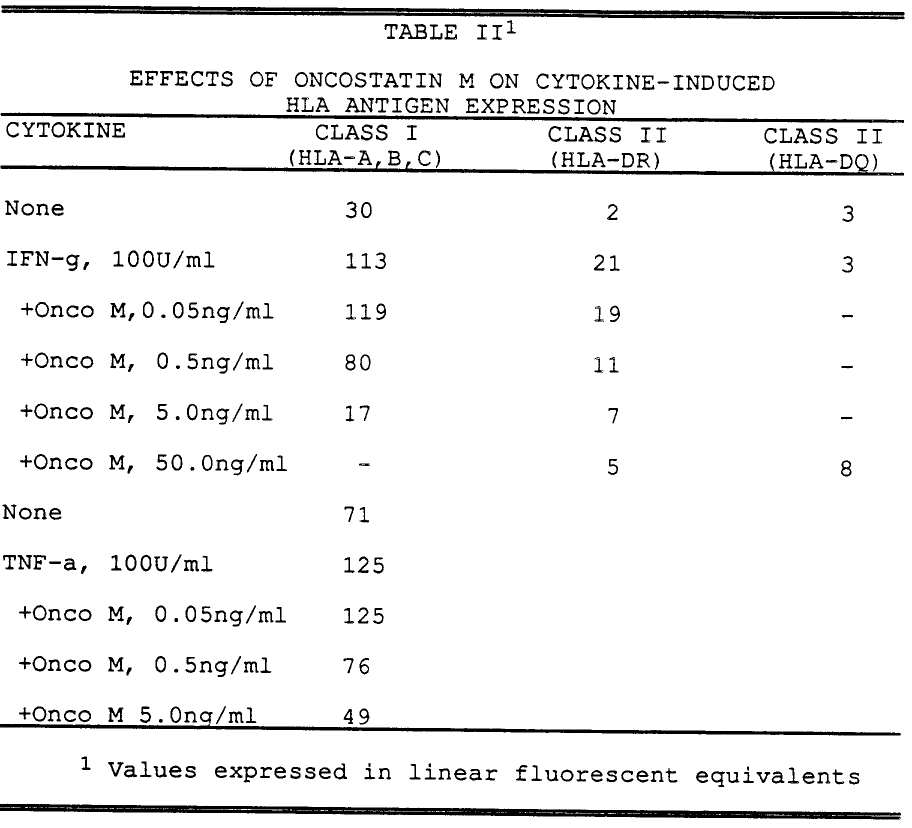

### 6.2.2. SYNERGISTIC ACTION OF ONCOSTATIN M AND TGF-β

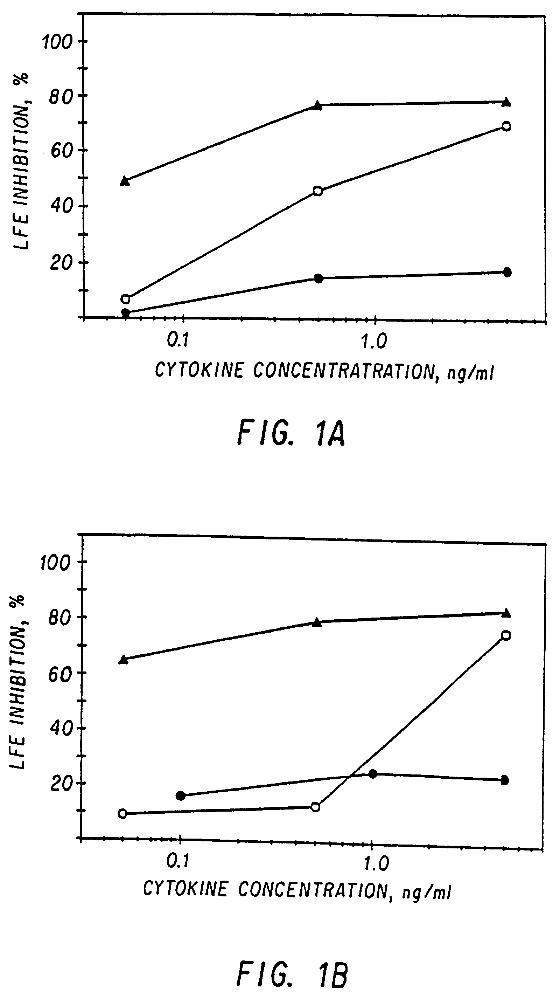

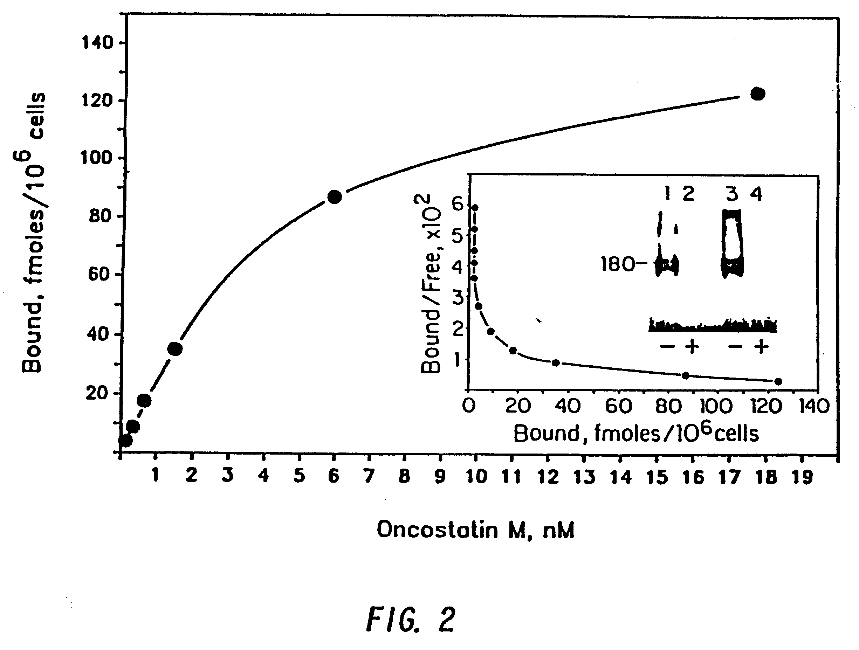

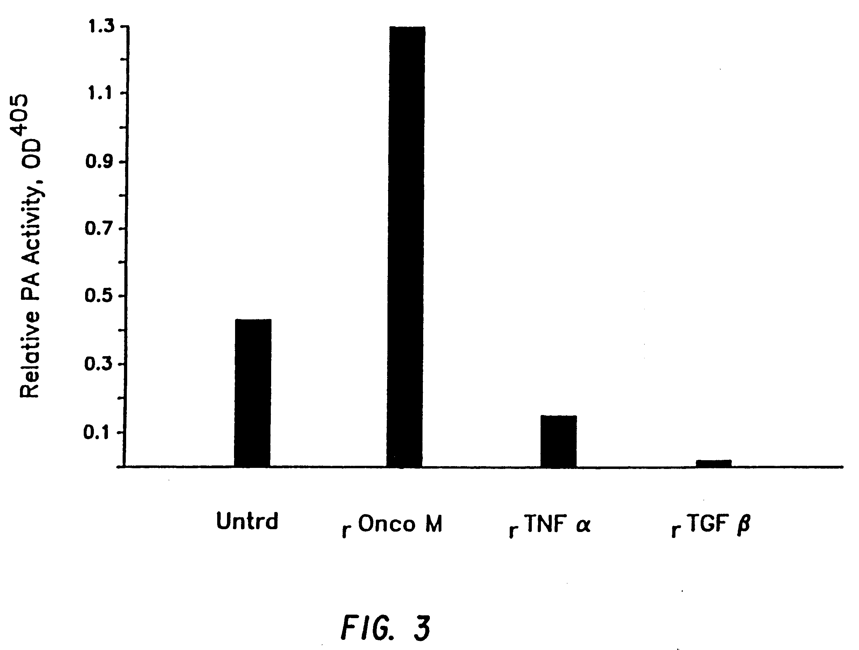

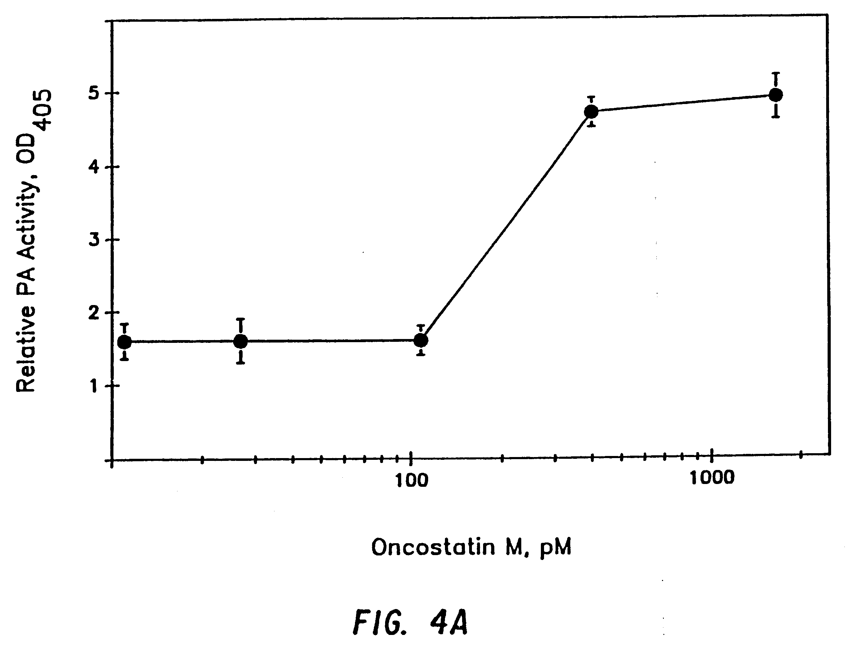

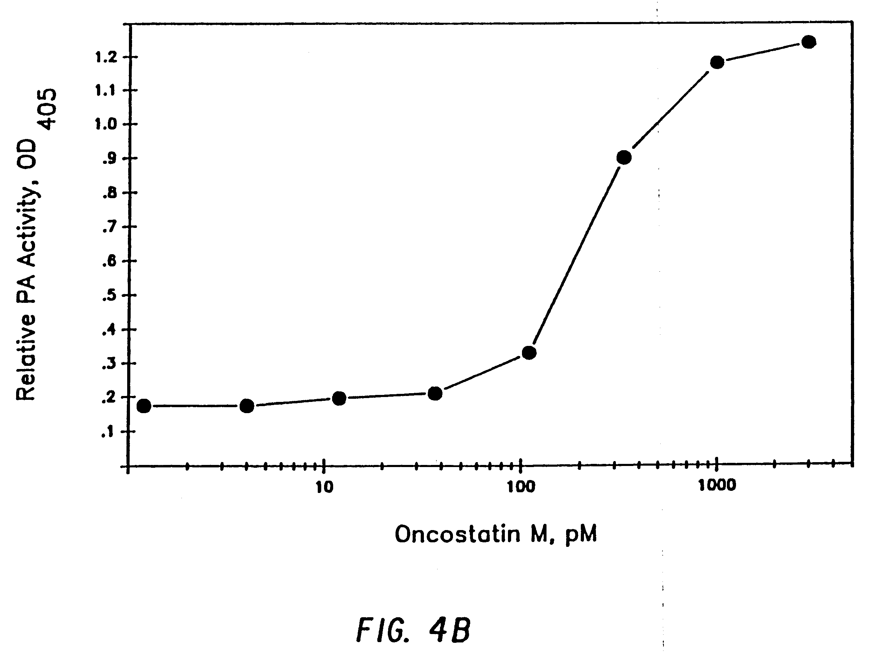

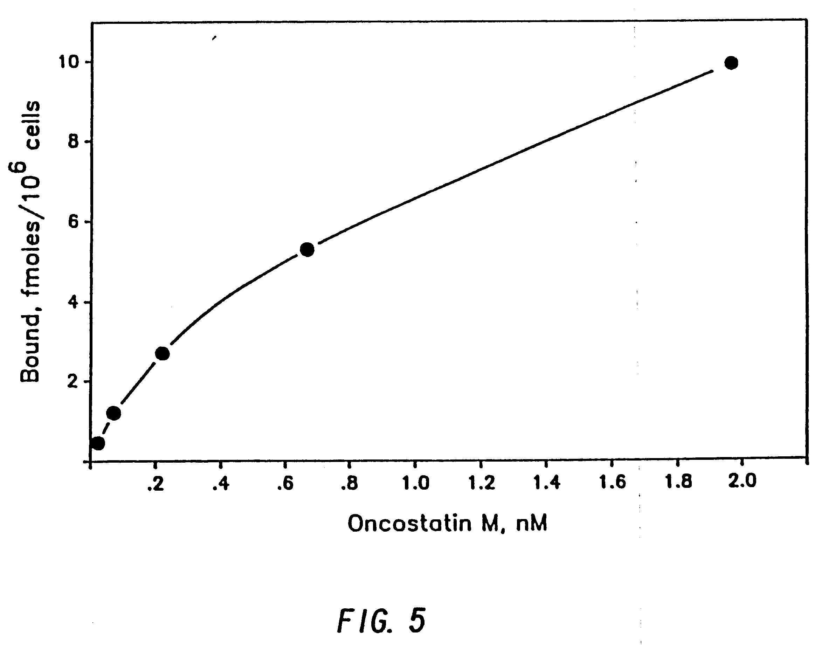

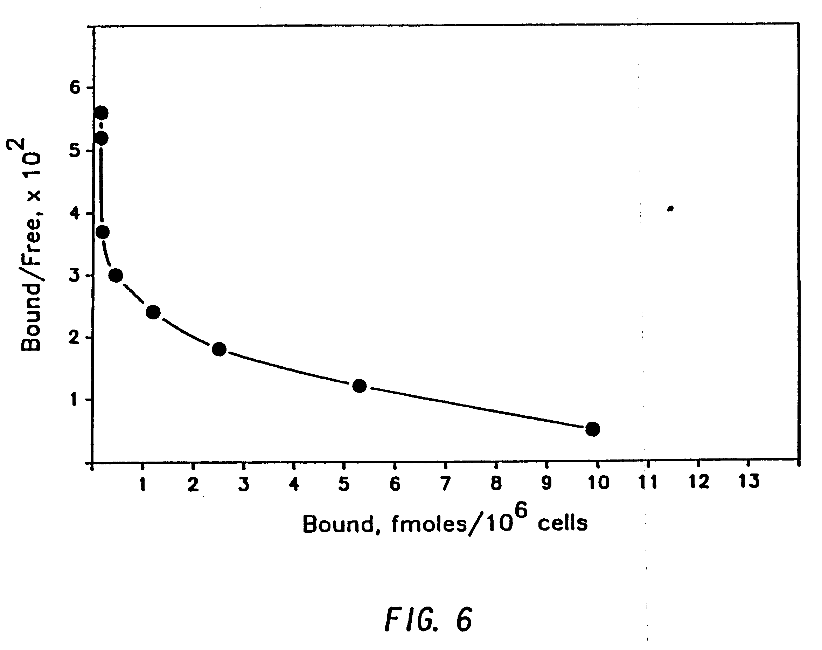

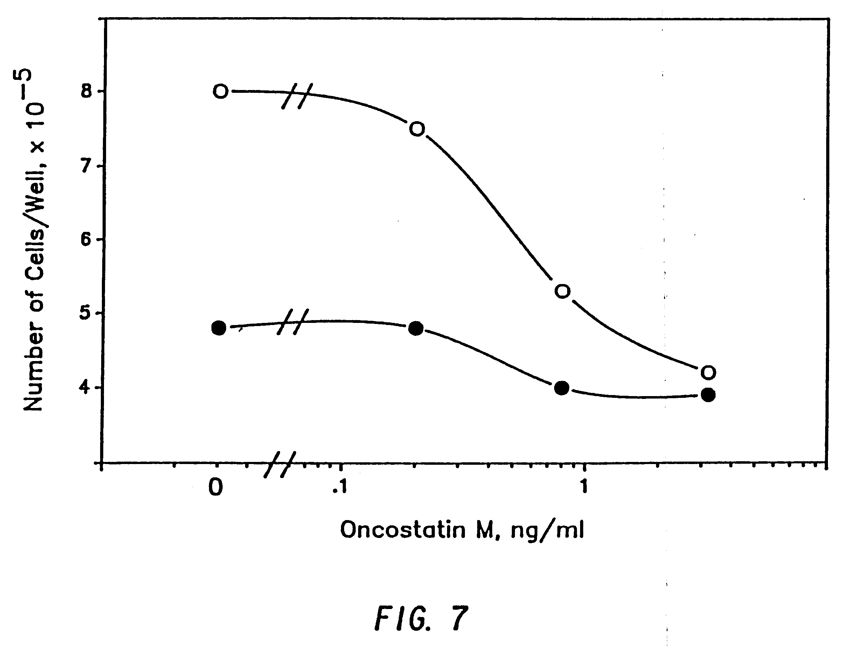

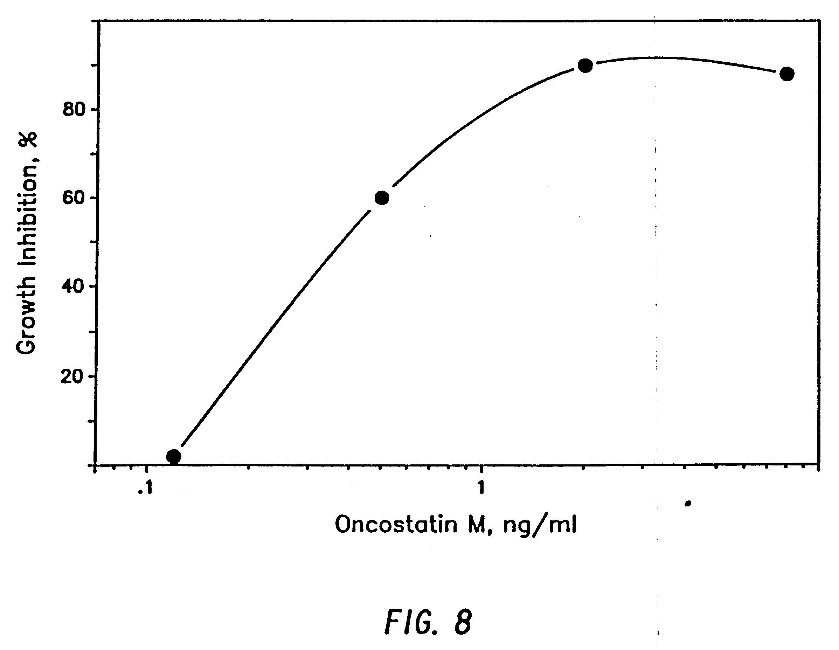

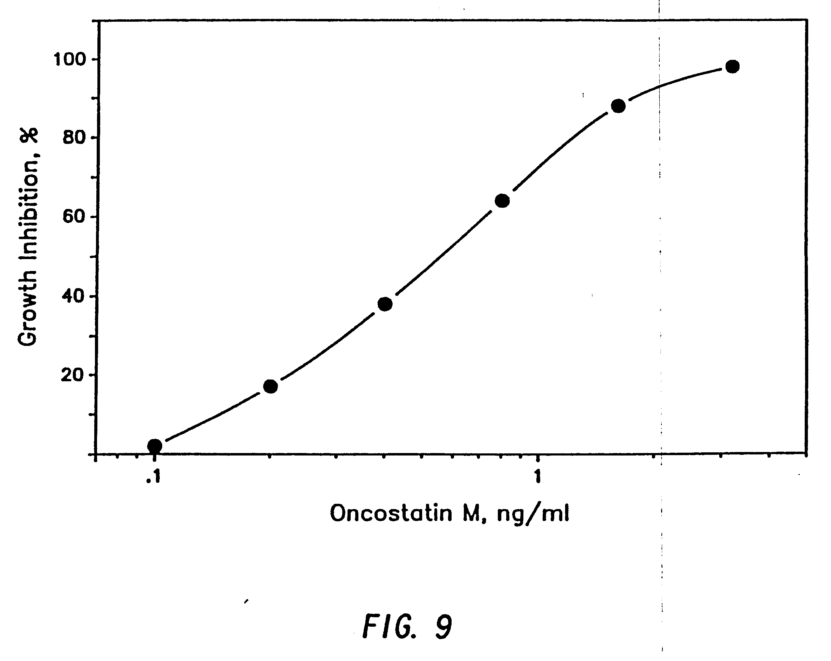

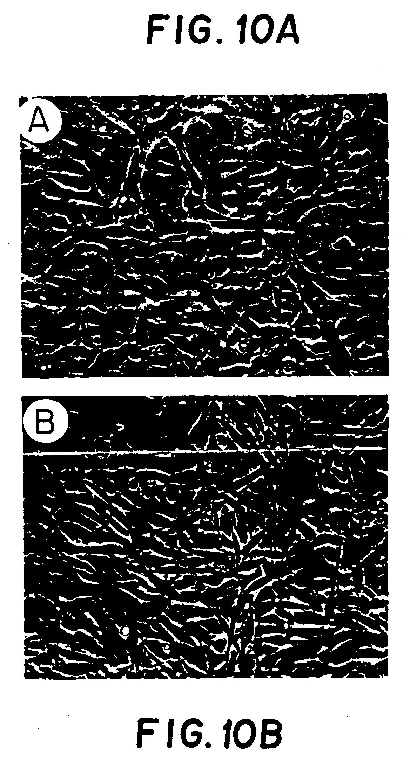

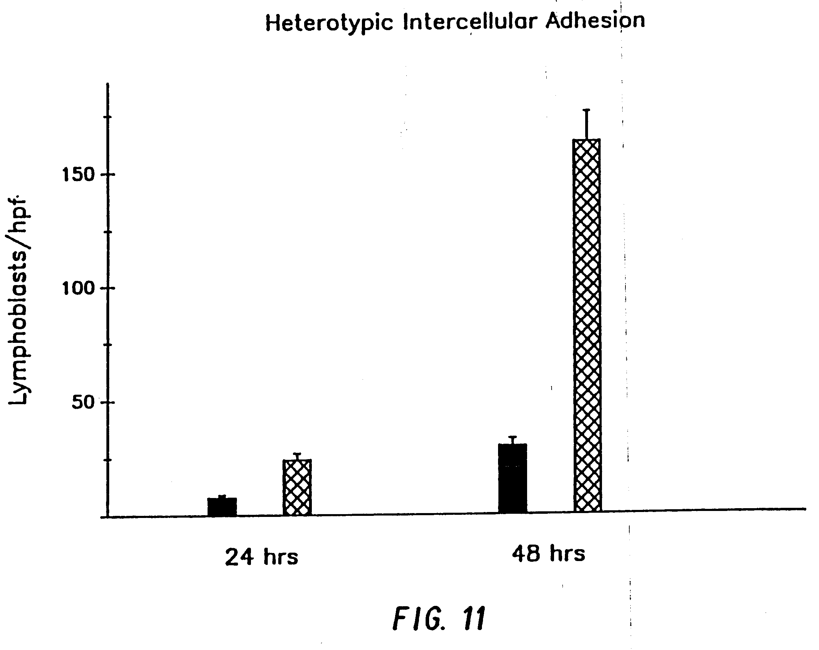

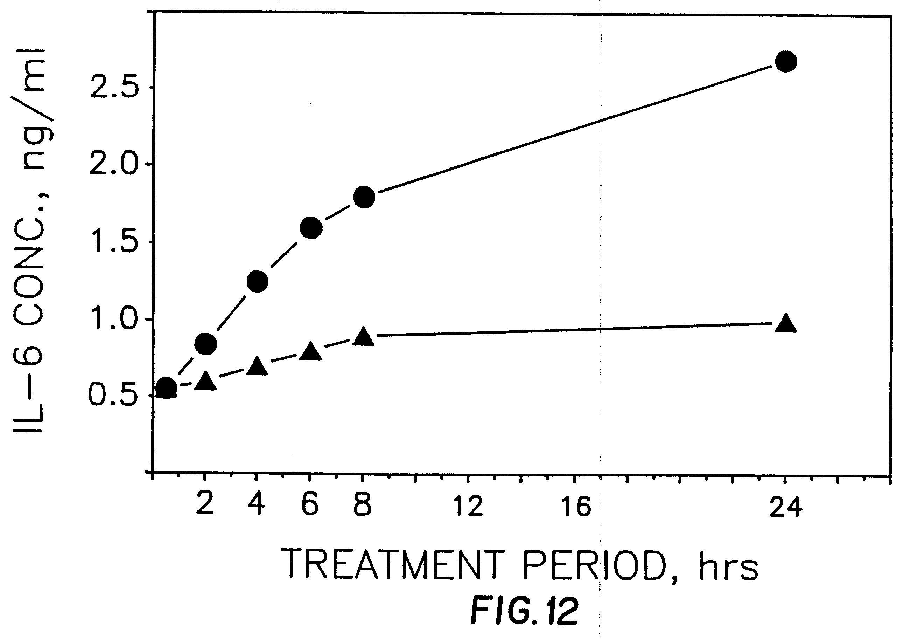

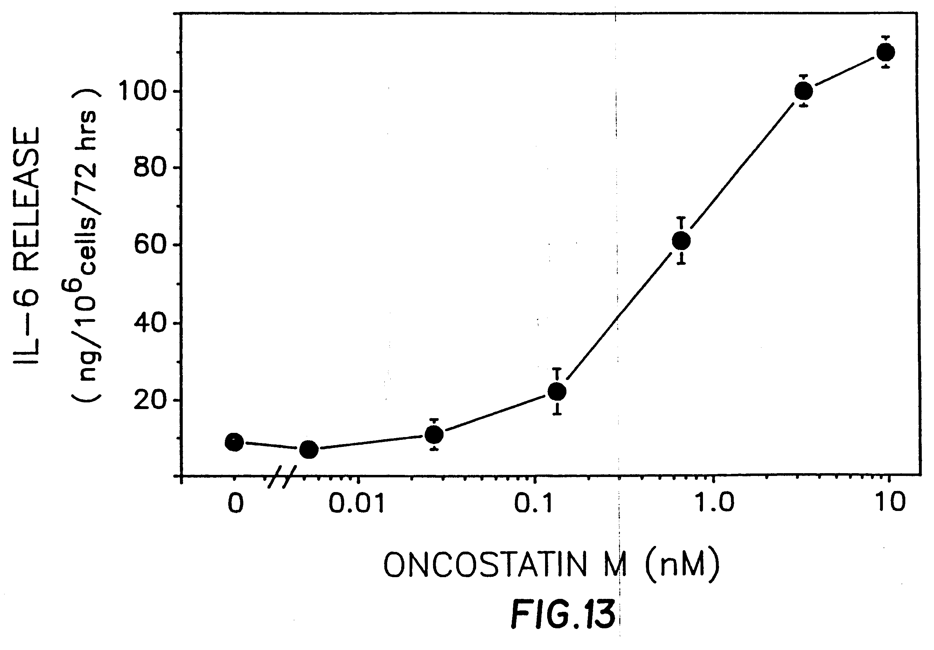

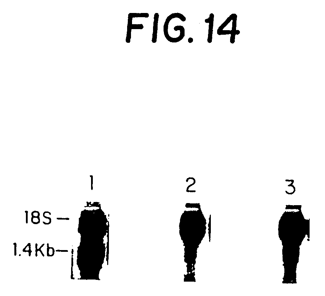

The effects of Oncostatin M, TGF-β, and Oncostatin M/TGF-β on IFN-γ-treated endothelial cells were evaluated and compared in order to determine whether synergistic action between TGF-β and Oncostatin M exists. The results of these experiments are illustrated in FIG. 1. TGF-β alone demonstrates a weak capacity for inhibiting IFN-γ-stimulated expression of either class I HLA-A,B,C or class II HLA-DR antigens. However, suboptimal amounts of Oncostatin M in combination with TGF-β resulted in synergistic antagonism of class I and class II antigen expression. For example, 50 pg/ml Oncostatin M and 0.5 ng/ml TGF-β in combination resulted in a 49% inhibition of class I antigen expression whereas independent treatment with the same concentration of Oncostatin M or TGF-β resulted in 7% and 15% inhibition respectively (FIG. 1A). Similarly, the combination of 0.5 ng/ml Oncostatin M and 1 ng/ml TGF-β resulted in an 80% inhibition of class II HLA-DR expression whereas independent treatments at these concentrations resulted in 13%(Oncostatin M) and 26%(TGF-β) inhibitions (FIG. 1B). Therefore, with respect to the inhibition of both class I and class II antigen expression, a synergistic effect some two times greater than the predicted additive effect was observed, suggesting compositions containing both TGF-β and Oncostatin M may be particularly useful.

### 6.2.3. TISSUE SPECIFICITY OF ONCOSTATIN M EFFECT ON HLA EXPRESSION

In order to explore whether the inhibitory effect of Oncostatin M on HLA antigen expression is specific for endothelial cells, the capacity of Oncostatin M to antagonize the expression of cytokine-induced class II MHC antigen expression on monocytes and macrophage-like cells was determined.

Human monocytes were isolated from blood using Ficoll gradients and adhered to plastic culture dishes. Cells were assayed for class II antigen as described in Materials and Methods. Cells were treated with 100 and 250 U/ml IFN-γ to boost basal levels of class II HLA antigens and parallel cultures were also treated with 5 ng/ml Oncostatin M. After a 3 day incubation, cells were stained with fluorescein-labeled antibodies and analyzed by indirect immunofluorescence and FACS quantitation as described in Materials and Methods, supra. The results indicate that Oncostatin M has no effect on either the basal or cytokine-induced levels of class II HLA antigen expression.

In another experiment, the effects of 1-10 ng/ml recombinant Oncostatin M on class II MHC antigen expression in a murine macrophage-like cell line (Wehi-3 cells) were investigated similarly. Class II antigen expression is strongly induced by either IFN-γ or TNF-α in these cells (Chang and Lee, 1968, J. Immunol. 137: 2853). Similarly, in the case of human monocytes, Oncostatin M had no effect on the basal or cytokine-induced levels of class II HLA antigen expression in these cells.

Since these results suggest that Oncostatin M is incapable of inhibiting class II HLA antigen expression on cells of the monocyte/macrophage lineage, Oncostatin M may offer a therapeutically more specific alternative to the broad immunosuppressive effects induced by other compounds such as Cyclosporin A, corticosteroids, and cyclophosphamides.

### 6.2.4. HIGH LEVEL EPXRESSION OF ONCOSTATIN M RECEPTORS ON HUVECs

HUVECs were grown to confluence in 24-well tissue culture dishes and radioreceptor assays conducted as described in Section 6.1.5., supra. FIG. 2 presents the saturation curve and Scatchard plot (inset) for Oncostatin M binding to HUVECs. Binding is saturable and the Scatchard plot shows a curvilinear isotherm indicating the presence of at least two classes of cell-surface binding sites on HUVECs. When analyzed by the two site model, a total concentration of 380,000 Oncostatin M receptor binding sites per cell were calculated, comprising 1,700 high affinity sites (Kd=5.6 pM) and 378,300 low affinity sites (Kd=8.5 nM). Half-maximal receptor occupancy occurred at 2 ng/ml (67 pM) which correlates with the ED₅₀ of Oncostatin M inhibition of cytokine-induced HLA antigen expression (Section 6.2.1., supra). These human endothelial cells appear to express approximately ten times more Oncostatin M receptors than bovine endothelial cells.

The Oncostatin M receptor on HUVECs was further characterized by chemically cross-linking [¹²⁵I]-Oncostatin M to its receptor and analyzing the structural properties of the complex by polyacrylamide gel electrophoresis as described (Linsley et al., 1989, J. Biol. Chem. 264: 4282-4289). As shown in the autoradiograph in FIG. 2, cross-linked ligand-receptor complex migrated on a 6% SDS-PAGE under reducing conditions as a prominent 180,000 molecular weight band (lane 3). The inclusion of 400-fold molar excess of unlabeled Oncostatin M during the binding period specifically competed out the radioactivity associated with this band (lane 4). Therefore, applicants conclude that the Oncostatin M receptor on human endothelial cells is similar in structure and function to the Oncostatin M receptor on bovine endothelial cells, as well as to the Oncostatin M receptor on human cells of non-endothelial origin (lanes 1 and 2).

## Claims

1. Use of Oncostatin M for the manufacture of a medicament for inhibiting endothelial tissue immunogenicity, whereby the expression of MHC antigens on the endothelial cells is suppressed.

2. The use of Oncostatin M according to claim 1, wherein the MHC antigens comprise class I HLA antigens.

3. The use of Oncostatin M according to claim 1, wherein the MHC antigens comprise class II HLA antigens.

4. The use of Oncostatin M according to any one of claims 1 to 3, wherein the Oncostatin M is covalently coupled to an antibody molecule that defines a cellular antigen.

5. The use of Oncostatin M according to any one of claims 1 to 3, wherein the Oncostatin M is covalently coupled to an hormone.

6. The use of Oncostatin M according to any one of claims 1 to 3, wherein the Oncostatin M is covalently coupled to a growth factor.

7. The use of Oncostatin M according to any one of claims 1 to 3, wherein the Oncostatin M is covalently coupled to a cytokine.

8. The use of Oncostatin M according to any one of claims 1 to 3, wherein the Oncostatin M is in a form to be co-administered with an effective amount of TGF-β.

9. The use of Oncostatin M according to any one of claims 1 to 8, wherein the Oncostatin M is encapsulated in a liposome.

10. The use of Oncostatin M according to any one of claims 1 to 8, wherein the Oncostatin M is encapsulated in a microcapsule.

11. Use of Oncostatin M for the manufacture of a medicament for improving the likelihood of transplanted organ functional success, whereby the expression of MHC antigens in donor organ vasculature is inhibited.

12. The use of Oncostatin M according to claim 11, wherein the transplanted organ is a heart.

13. The use of Oncostatin M according to claim 11, wherein the transplanted organ is a lung.

14. The use of Oncostatin M according to claim 11, wherein the transplanted organ is a kidney.

15. The use of Oncostatin M according to claim 11, wherein the transplanted organ is a liver.

16. The use of Oncostatin M according to any one of claims 11 to 15, wherein the MHC antigens comprise class I HLA antigens.

17. The use of Oncostatin M according to any one of claims 11 to 15, wherein the MHC antigens comprise class II HLA antigens.

18. The use of Oncostatin M according to any one of claims 11 to 17, wherein the Oncostatin M is covalently coupled to an antibody molecule that defines a cellular antigen.

19. The use of Oncostatin M according to any one of claims 11 to 17, wherein the Oncostatin M is covalently coupled to an hormone.

20. The use of Oncostatin M according to any one of claims 11 to 17, wherein the Oncostatin M is covalently coupled to a growth factor.

21. The use of Oncostatin M according to any one of claims 11 to 17, wherein the Oncostatin M is covalently coupled to a cytokine.

22. The use of Oncostatin M according to any one of claims 11 to 17, wherein the Oncostatin M is in a form to be co-administered with an effective amount of TGF-β.

23. The use of Oncostatin M according to any one of claims 11 to 22, wherein the Oncostatin M is encapsulated in a liposome.

24. The use of Oncostatin M according to any one of claims 11 to 22, wherein the Oncostatin M is encapsulated in a microcapsule.

## Patentansprüche

1. Verwendung von Oncostatin M für die herstellung vn einem Medikament zur Hemmung der Immunogenizität von endothelialen Gewebe, wodurch der Ausdruck von MHC Antigenen auf den endothelialen Zellen unterdrückt wird.

2. Die Verwendung von Oncostatin M gemäß Anspruch 1, wobei die MHC-Antigene HLA-Antigene der Klasse I umfassen.

3. Die Verwendung von Oncostatin M gemäß Anspruch 1, wobei die MHC-Antigene HLA-Antigene der Klasse II umfassen.

4. Die Verwendung von Oncostatin M gemäß einem der vorgehenden Ansprüche 1 bis 3, wobei das Oncostatin M kovalent an ein Antikörpermolekül gekoppelt ist, das ein zelluläres Antigen darstellt.

5. Die Verwendung von Oncostatin M gemäß einem der vorgehenden Ansprüche 1 bis 3, wobei das Oncostatin M kovalent an ein Hormon gekoppelt ist.

6. Die Verwendung von Oncostatin M gemäß einem der vorgehenden Ansprüche 1 bis 3, wobei das Oncostatin M kovalent an einen Wachstumsfaktor gekoppelt ist.

7. Die Verwendung von Oncostatin M gemäß einem der vorgehenden Ansprüche 1 bis 3, wobei das Oncostatin M kovalent an ein Zytokin gekoppelt ist.

8. Die Verwendung von Oncostatin M gemäß einem der vorgehenden Ansprüche 1 bis 3, wobei das Oncostatin M in einer Form für die gemeinsame Verabreichung mit einer wirksamen Menge von TGF-β ist.

9. Die Verwendung von Oncostatin M gemäß einem der vorgehenden Ansprüche 1 bis 8, wobei das Oncostatin M in einem Liposom verkapselt ist.

10. Die Verwendung von Oncostatin M gemäß einem der vorgehenden Ansprüche 1 bis 8, wobei das Oncostatin M in einer Mikrokapsel verkapselt ist.

11. Verwendung von Oncostatin M für die Herstellung von eineme Medikament für die Verbesserung der Wahrscheinlichkeit eines funktionellen Erfolgs von transplantierten Organen, wodurch die Expression von MHC-Antigenen in dem Gefäßsystem des Spenderorgans gehemmt wird.

12. Die Verwendung voll Oncostatin M gemäß Anspruch 11, wobei das transplantierte Organ ein Herz ist.

13. Die Verwendung von Oncostatin M gemäß Anspruch 11, wobei das transplantierte Organ eine Lunge ist.

14. Die Verwendung von Oncostatin M gemäß Anspruch 11, wobei das transplantierte Organ eine Niere ist.

15. Die Verwendung von Oncostatin M gemäß Anspruch 11, wobei das transplantierte Organ eine Leber ist.

16. Die Verwendung von Oncostatin M gemäß einem der Ansprüche 11 bis 15, wobei die MHC-Antigene HLA-Antigene der Klasse I umfassen.

17. Die Verwendung von Oncostatin M gemäß einem der Ansprüche 11 bis 15, wobei die MHC-Antigene HLA-Antigene der Klasse II umfassen.

18. Die Verwendung von Oncostatin M gemäß einem der Ansprüche 11 bis 17, wobei das Oncostatin M kovalent an ein Antikörpermolekül gekoppelt ist, das ein zelluläres Antigen darstellt.

19. Die Verwendung von Oncostatin M gemäß einem der Ansprüche 11 bis 17, wobei das Oncostatin M kovalent an ein Hormon gekoppelt ist.

20. Die Verwendung von Oncostatin M gemäß einem der Ansprüche 11 bis 17, wobei das Oncostatin M kovalent an einen Wachstumsfaktor gekoppelt ist.

21. Die Verwendung von Oncostatin M gemäß einem der Ansprüche 11 bis 17, wobei das Oncostatin M kovalent an ein Zytokin gekoppelt ist.

22. Die Verwendung von Oncostatin M gemäß einem der Ansprüche 11 bis 17, wobei das Oncostatin M in einer Form für die gemeinsame Verabreichung mit einer wirksamen Menge von TGF-β ist.

23. Die Verwendung von Oncostatin M gemäß einem der Ansprüche 11 bis 17, wobei das Oncostatin M in einem Liposom verkapselt ist.

24. Die Verwendung von Oncostatin M gemäß einen der Ansprüche 11 bis 17, wobei das Oncostatin M in einer Mikrokapsel verkapselt ist.

## Revendications

1. Utilisation d'oncostatine M pour la préparation d'un médicament pour l'inhibition de l'immunogénicité de tissus endothéliaux, ce par quoi l'expression d'antigènes MHC sur les cellules endothéliales est supprimée.

2. Utilisation d'oncostatine M selon la revendication 1, dans laquelle les antigènes MHC comprennent les antigènes HLA de la classe I.

3. Utilisation d'oncostatine M selon la revendication 1, dans laquelle les antigènes MHC comprennent les antigènes HLA de la classe II.

4. L'utilisation d'oncostatine M selon une quelconque des revendications 1 à 3, dans laquelle l'oncostatine M est associée de manière covalente à une molécule d'anticorps qui définit un antigène cellulaire.

5. L'utilisation d'oncostatine M selon une quelconque des revendications 1 à 3, dans laquelle l'oncostatine M est associée de manière covalente à une hormone.

6. L'utilisation d'oncostatine M selon une quelconque des revendications 1 à 3, dans laquelle l'oncostatine M est associée de manière covalente à un facteur de croissance.

7. L'utilisation d'oncostatine M selon une quelconque des revendications 1 à 3; dans laquelle l'oncostatine M est associée de manière covalente à une cytokine.

8. L'utilisation d'oncostatine M selon une quelconque des revendications 1 à 3, dans laquelle l'oncostatine M est une forme destinée à être administrée en association avec une quantité efficace de TGF-β.

9. L'utilisation d'oncostatine M selon une quelconque des revendications 1 à 8, dans laquelle l'oncostatine M est encapsulée dans un liposome.

10. L'utilisation d'oncostatine M selon une quelconque des revendications 1 à 8, dans laquelle l'oncostatine M est encapsulée dans une microcapsule.

11. Utilisation d'oncostatine M pour la préparation d'un médicament pour l'amélioration des chances de réussite fonctionnelle d'un organe transplanté, ce par quoi l'expression d'antigènes MHC dans le système vasculaire de l'organe donneur est inhibée.

12. L'utilisation d'oncostatine M selon la revendication 11, dans laquelle l'organe transplanté est le coeur.

13. L'utilisation d'oncostatine M selon la revendication 11 dans laquelle l'organe transplanté est le poumon.

14. L'utilisation d'oncostatine M selon la revendication 11, dans laquelle l'organe transplanté est le rein.

15. L'utilisation d'oncostatine M selon la revendication 11, dans laquelle l'organe transplanté est le foie.

16. L'utilisation d'oncostatine M selon une quelconque des revendications 11 à 15, dans laquelle les antigènes MHC comprennent les antigènes HLA de la classe I.

17. L'utilisation d'oncostatine M selon une quelconque des revendications 11 à 15, dans laquelle les antigènes MHC conprennent les antigènes HLA de la classe II.

18. L'utilisation d'oncostatine M selon une quelconque des revendications 11 à 17, dans laquelle l'oncostatine M est associée de manière covalente à une molécule d'anticorps qui définit un antigène cellulaire.

19. L'utilisation d'oncostatine M selon une quelconque des revendications 11 à 17, dans laquelle l'oncostatine M est associée de manière covalente à une hormone.

20. L'utilisation d'oncostatine M selon une quelconque des revendications 11 à 17, dans laquelle l'oncostatine M est associée de manière covalente à un facteur de croissance.

21. L'utilisation d'oncostatine M selon une quelconque des revendications 11 à 17, dans laquelle l'oncostatine M est associée de manière covalente à une cytokine.

22. L'utilisation d'oncostatine M selon une quelconque des revendications 11 à 17, dans laquelle l'oncostatine M est sous une forme destinée à être administrée en association avec une quantité efficace de TGF-β.

23. L'utilisation d'oncostatine M selon une quelconque des revendications 11 à 22, dans laquelle l'oncostatine M est encapsulée sans un liposome.

24. L'utilisation d'oncostatine M selon une quelconque des revendications 11 à 22, dans laquelle l'oncostatine M est encapsulée dans une microcapsule.
